(19) **Europäisches Patentamt**
**European Patent Office**
**Office européen des brevets**

(11) **EP 4 752 758 A1**

(12) **EUROPEAN PATENT APPLICATION**
published in accordance with Art. 153(4) EPC

(43) Date of publication:
03.06.2026 Bulletin 2026/23

(51) International Patent Classification (IPC):
*G06F 17/15* (2006.01)     *G06Q 10/04* (2023.01)
*G16B 40/00* (2019.01)

(21) Application number: 24845166.8

(22) Date of filing: 21.05.2024

(52) Cooperative Patent Classification (CPC):
G06F 17/15; G06Q 10/04; G16B 40/00

(86) International application number:
PCT/JP2024/018720

(87) International publication number:
WO 2025/022782 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 25.07.2023 JP 2023120928

(71) Applicant: Japan Science and Technology
Agency
Kawaguchi-shi, Saitama 332-0012 (JP)

(72) Inventors:
• CHEN Luonan
  Tokyo 113-0033 (JP)
• AIHARA Kazuyuki
  Tokyo 113-0033 (JP)
• LIU Rui
  Guangzhou-city, Guangdong 510640 (CN)
• CHEN Pei
  Guangzhou-city, Guangdong 510640 (CN)

(74) Representative: Noble, Nicholas et al
Kilburn & Strode LLP
Lacon London
84 Theobalds Road
London WC1X 8NL (GB)

(54) **SIGN DETECTION DEVICE, SIGN DETECTION METHOD, PROGRAM, AND RECORDING MEDIUM**

(57) An predictive sign detection device 1 for detecting a predictive sign of a transition in the state of a target, comprises an acquisition unit 10 that acquires spatiotemporal data of multidimensional variables indicating the state of the targe, a prediction error analyzer 20 that estimates the deviation between the predicted value and the measured value of the time data in the spatiotemporal data, a fluctuation analyzer 30 that estimates a fluctuation in spatiotemporal data and a predictive sign detector 40. The prediction error analyzer 20 calculates the predicted value by converting the spatiotemporal data of multidimensional variables into the time data of the target variable, which is the variable to be predicted by the predictive sign detection device, and estimates the deviation between the predicted value and the measured value. The fluctuation analyzer 30 calculates the fluctuation of the target state by fluctuation analysis using the spatiotemporal data. The predictive sign detector 40 detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

EP 4 752 758 A1

# FIG. 1

SPATIOTEMPORAL DATA

1

PREDICTIVE SIGN DETECTION DEVICE                    10

ACQUISITION UNIT

SPATIOTEMPORAL DATA          20          SPATIOTEMPORAL DATA          30

PREDICTION ERROR ANALYZER          FLUCTUATION ANALYZER

DEVIATION BETWEEN PREDICTED VALUE AND MEASURED VALUE OF SPATIOTEMPORAL DATA          FLUCTUATION OF SPATIOTEMPORAL DATA          40

PREDICTIVE SIGN DETECTOR

PREDICTIVE SIGN

**Description**

TECHNICAL FIELD

This disclosure relates to a predictive sign detection device, predictive sign detection method, program and recording medium.

BACKGROUND ART

In natural phenomena, such as occurrence of earthquakes or onset of disease, and social phenomena, such as large fluctuations in stock prices, it is known that a sudden transition from one stable state to another state occurs when a target state reaches a certain critical point, hereinafter referred to as "bifurcation point" or "tipping point", see, for example, Non-Patent Literature 1-5. In these cases, it is expected to be possible to cope with rapid changes in natural and social phenomena if it is possible to detect a predictive sign of a rapid state transition.

In particular, with regard to research on the dynamic mechanism of complex diseases, it is known that some researchers have found a relationship between the state transition at bifurcation points and rapid deterioration of diseases by modeling the process of disease

deterioration ,e.g., asthma attacks, cancer development and recurrence, as a time-dependent nonlinear dynamical system and analyzing this system, see, for example, Non-Patent Literature 6.

Against this background, a technology for detecting candidates for a dynamical network biomarker (Dynamical Network Biomarker, hereinafter also referred to as "DNB"), which is an indicator of symptoms of a living body, has been disclosed, see, for example, Patent Literature 1. DNB is based on measurement data for several factor items obtained from measurements on living organisms.

Furthermore, in the transition state from a normal state to a diseased state, there is disclosed a technology to detect a pre-disease state based on local network entropy by considering the state transition of a factor of interest and a connecting factor directly and dynamically connected to this factor as the local network entropy, see, for example, Patent Literature 2. Furthermore, there is disclosed a technology to determine whether DNB is occurring or not by storing the data of biological substances collected and analyzed from multiple healthy humans and adding the analysis data of biological substances collected only once from a subject to it, see, for example, Patent Literature 3.

RELATED-ART LITERATURE

PATENT LITERATURES

Patent Literature 1: JP 2014-64515
Patent Literature 2: JP 2014-83194
Patent Literature 3: WO 2018/207925

NON PATENT LITERATURES

Non Patent Literature 1: Venegas, J.G., Winkler, T., Musch, G., Melo, M.F.V., Layfield, D., Tgavalekos, N., Fischman, A.J., Callahan, R.J., Bellani, G., and Harris, R. S., "Self-organized patchiness in asthma as a prelude to catastrophic shifts," Nature 434, pp. 777-782 (2005).
Non Patent Literature 2: McSharry, P.E., Smith, L.A., and Tarassenko, L., "Prediction of epileptic seizures: are nonlinear methods relevant? Nature Medicine 9, pp. 241-242 (2003) .
Non Patent Literature 3: Barriuso, R.P., Guallar, E., and Coresh, J., "Transition models for change-point estimation in logistic regression " Statistics in Medicine 22(7), pp. 1141-1162 (2003).
Non Patent Literature 4: Paek, S.H., Chung, H.-T., Jeong, S.S., Park, C.-K., Kim, C.-Y, Kim, J.E., Kim D.G., and Jung, H.-W., "Hearing preservation after gamma knife stereotactic radiosurgery of vestibular schwannoma," Cancer 104, pp. 580-590 (2005).
Non Patent Literature 5: Liu, J.K., Rovit, R.L., and Couldwell, W.T., "Pituitary Apoplex
Seminars in Neurosurgery 12, Thieme, pp. 315-320 (2001).
Non Patent Literature 6: Tanaka, G., Tsumoto, K., Tsuji, S., and Aihara, K., "Bifurcation analysis on a hybrid systems model of intermittent hormonal therapy for prostate cancer," Physica Dermatol. Physica D: Nonlinear Phenomena Volume 237, pp. 2616-2627 (2008).
Non Patent Literature 7: Takens, F., "Dynamical Systems and Turbulence. "In: Rand, D. and Young, L.S., Eds.,

Lecture Notes in Mathematics. Vol. 898, pp. 366-381, (1981)

Non Patent Literature 8: Sauer, T., Yorke, J.A., and Casdagli, M., "Embedology," J. Stat. Phys. 65, pp. 579-615 (1991).

Non Patent Literature 9: Sauer, T., Yorke, J.A., and Casdagli, M., "Embedology," J. Stat. Phys. 65, pp. 579-615 (1991).

Non Patent Literature 10: Chen, L., Liu, R., Liu, Z.-P., Li, M., and Aihara K., "Detecting early-warning signals for sudden deterioration of complex diseases by dynamical network biomarkers" Scientific Reports volume 2, Article number: 342 (2012)

TECHNICAL PROBLEM

The techniques disclosed in the Patent Literatures 1-3 can achieve improvement of resistance to noise in the detection of a predictive sign and simplification of the sample collection required for the detection of the predictive sign. However, there is still room for further study with regard to the precise timing and accuracy of the determination of a predictive sign.

The purpose of this disclosure is to accurately detect the timing of a predictive sign when the state of the target transition.

SOLUTION TO PROBLEM

In order to solve the above problem, a predictive sign detection device according to an aspect of the disclosure is a device for detecting a predictive sign of a transition in the state of a target. The device comprises an acquisition unit that acquires spatiotemporal data of multidimensional variables indicating the state of the target, a prediction error analyzer that estimates the deviation between the predicted value and the measured value of the time data in the spatiotemporal data, a fluctuation analyzer that estimates a fluctuation in spatiotemporal data and a predictive sign detector. The prediction error analyzer calculates the predicted value by converting the spatiotemporal data of multidimensional variables into the time data of the target variable, which is the variable to be predicted by the predictive sign detection device, and estimates the deviation between the predicted value and the measured value. The fluctuation analyzer calculates the fluctuation of the target state by fluctuation analysis using the spatiotemporal data. The predictive sign detector detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

In one embodiment, the transformation from spatiotemporal data to time data of the target variable may be a transformation by embedding in non-time-delayed coordinate space or in time-delayed coordinate space.

In one embodiment, the transformation from spatiotemporal data to time data of the target variable may be computed using machine learning.

In one embodiment, the fluctuation analyzer may calculate the fluctuation of the state of the target by converting to time data of virtual variables based on spatiotemporal data.

In one embodiment, the virtual variables may be data representing the movement of the target state on the central manifold.

In one embodiment, the fluctuation analyzer may estimate the fluctuation of spatiotemporal data using the dynamic network markers.

In one embodiment, the fluctuation analyzer may calculate fluctuation when the deviation estimated by the prediction error analyzer exceeds a predetermined first threshold value.

In one embodiment, the transition of the state of the target may be the occurrence of an earthquake.

In one embodiment, the prediction sign of when the state of the subject will transition may be the occurrence of a pre-disease state, which is a state of transition from a normal state to a disease state.

In one embodiment, the transformation from non-time-delayed coordinate space to time-delayed coordinate space may be expressed as follows,

$$\Phi(Z^t) = V^-,$$

$$Z^t = \Psi(V^t),$$

$$\Phi\Psi = I.$$

Z is a vector at time t in non-time-delayed coordinate space, V is a vector at time t in time-delayed coordinate

space, $\Phi$ and $\Psi$ are nonlinear transformations and I is an identity transformation.

In one embodiment, the transformation from non-time-delayed coordinate space to time-delayed coordinate space may be expressed as follows,

$$PF(Z^L) = V^L,$$

$$F(Z^t) = QV^t,$$

$$PQ = E.$$

F is a nonlinear transformation, P and Q are weight matrices and E is a unit matrix.

Another aspect of the disclosure is a predictive sign detection method for detecting a predictive sign of a transition in the state of a target. The method comprises an acquisition step to acquire spatiotemporal data of multidimensional variables indicating the state of the target, a prediction error analysis step to estimate the deviation between the predicted value and the measured value of the time data, a fluctuation analysis step to estimate the fluctuation of the spatiotemporal data and a predictive sign detection step. The prediction error analysis step calculates the predicted value by converting the spatiotemporal data of multidimensional variables into the time data of the target variable, which is the variable to be predicted by the predictive sign detection device, and estimates the deviation between the predicted value and the measured value. The fluctuation analysis step calculates the fluctuation of the target state by fluctuation analysis using the spatiotemporal data. The predictive sign detection step detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

Yet another aspect of the invention is a program for causing a computer to execute a method for detecting a predictive sign of a transition in the state of a target. The method comprises an acquisition step to acquire spatiotemporal data of multidimensional variables indicating the state of the target, a prediction error analysis step to estimate the deviation between the predicted value and the measured value of the time data, a fluctuation analysis step to estimate the fluctuation of the spatiotemporal data and a predictive sign detection step. The prediction error analysis step calculates the predicted value by converting the spatiotemporal data of multidimensional variables into the time data of the target variable, which is the variable to be predicted by the predictive sign detection device, and estimates the deviation between the predicted value and the measured value. The fluctuation analysis step calculates the fluctuation of the target state by fluctuation analysis using the spatiotemporal data. The predictive sign detection step detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

Yet another aspect of the invention is a recording medium containing a program for causing a computer to execute a method for detecting a predictive sign of a transition in the state of a target. The method comprises an acquisition step to acquire spatiotemporal data of multidimensional variables indicating the state of the target, a prediction error analysis step to estimate the deviation between the predicted value and the measured value of the time data, a fluctuation analysis step to estimate the fluctuation of the spatiotemporal data and a predictive sign detection step. The prediction error analysis step calculates the predicted value by converting the spatiotemporal data of multidimensional variables into the time data of the target variable, which is the variable to be predicted by the predictive sign detection device, and estimates the deviation between the predicted value and the measured value. The fluctuation analysis step calculates the fluctuation of the target state by fluctuation analysis using the spatiotemporal data. The predictive sign detection step detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

Any combination of the above components, and any transformation of the expression of the present invention among devices, methods, systems, recording media, computer programs, etc., is also valid as an aspect of the present disclosure.

ADVANTAGEOUS EFFECTS OF INVENTION

According to the present invention, it is possible to accurately detect the timing of a predictive sign when the state of the target transition.

BRIEF DESCRIPTION OF DRAWINGS

[FIG. 1] Fig. 1 shows a functional block diagram of a predictive sign detection device according to the first embodiment;

[FIG. 2] Fig. 2 shows a graph showing the time evolution of the predicted values and the measured values of spatiotemporal data;
[FIG. 3] Fig. 3 shows a graph showing the time evolution of the fluctuation in the spatiotemporal data;
[FIG. 4] Fig. 4 shows a graph of the timing of the detection of the predictive sign of the earthquake and the actual occurrence of the earthquake;
[FIG. 5] Fig. 5 shows a schematic diagram of the embedding of time-delayed coordinates;
[FIG. 6] Fig. 6 shows a schematic diagram showing the embedding of non-time-delayed coordinates;
[FIG. 7] Fig. 7 is a schematic diagram showing the vector of multidimensional spatiotemporal data and target variables;
[FIG. 8] Fig. 8 shows a schematic diagram of an autoreservoir neural network;
[FIG. 9] Fig. 9 shows a flowchart showing the processing steps of a predictive sign detection method according to the second embodiment;
[FIG. 10] Fig. 10 shows a schematic diagram explaining the definition of pre-disease state;
[FIG. 11] Fig. 11 shows a schematic diagram showing the integration of DNB theory analysis and prediction theory analysis.

DESCRIPTION OF EMBODIMENTS

The disclosure will be explained below with reference to each drawing based on a suitable embodiment. In the embodiments and variations, identical or equivalent components and members shall be marked with the same symbols, and redundant explanations will be omitted where appropriate. The dimensions of the components in each drawing are shown enlarged or reduced as appropriate for ease of understanding. In each drawing, some parts of the components that are not important in explaining the embodiment are omitted. In addition, terms including ordinal numbers such as first and second are used to describe various components, however these terms are used only to distinguish one component from other components, and the components are not limited by these terms.

The first embodiment

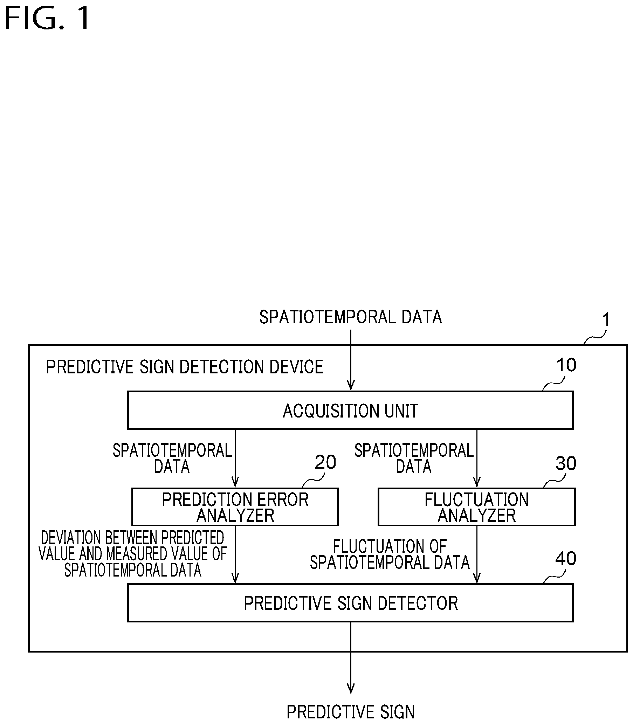

Figure 1 shows a functional block diagram of a predictive sign detection device 1 according to the first embodiment. The predictive sign detection device 1 is a device that detects a predictive sign of transition in the state of a target. The predictive sign detection device 1 comprises an acquisition unit 10, a prediction error analyzer 20, a fluctuation analyzer 30 and a predictive sign detector 40.
The acquisition unit 10 acquires spatiotemporal data of multidimensional variables indicating the state of a target. For example, if the target is a person, the spatiotemporal data is biometric data such as gene expression generated from a human biological sample. In this case, the predictive sign corresponds to the pre-disease state, which is the bifurcation point when a person transitions from a normal state to a diseased state. Or, for example, if the target is the earth, the spatiotemporal data is the movement of the earth's surface over a wide area acquired by the Global Navigation Satellite System, GNSS. In this case, the predictive sign corresponds to the state immediately before the transition from a normal state to an earthquake state.
The acquisition unit 10 transmits the acquired spatiotemporal data to the prediction error analyzer 20 and the fluctuation analyzer 30.
The prediction error analyzer 20 estimates the deviation between the predicted value and the measured value of the time data in the spatiotemporal data. Specifically, the prediction error analyzer 20 calculates the predicted value by converting the spatiotemporal data of multidimensional variables into the time data of the target variable, which is the variable to be predicted by the predictive sign detection device 1, and estimates the deviation between this predicted value and the measured value.
When the predicted value of the time data in the spatiotemporal data and the measured value is monitored for a certain period of time, the deviation between the two values may suddenly become large. The moment when this deviation becomes large is considered to be one of the indicators of a bifurcation point of a state transition. Therefore, a predictive sign of a state transition can be determined based on the fact that the deviation exceeds a certain value, i.e. threshold value. Further details of the method for estimating the deviation between the predicted value and the measured value will be explained later.
The fluctuation analyzer 30 estimates a fluctuation in the spatiotemporal data. Specifically, the fluctuation analyzer 30 calculates the fluctuation of the target state by fluctuation analysis using the spatiotemporal data.

When the spatiotemporal data is monitored for a certain period of time, a large fluctuation may suddenly occur in the spatiotemporal data. The moment when this large fluctuation occurs is also considered to be one of the indicators of a bifurcation point of a state transition. Therefore, a predictive sign of a state transition can be determined based on the fact that the fluctuation of the spatiotemporal data exceeds a certain value, threshold value. Further details of the method for calculating the fluctuation of the target state by fluctuation analysis will be explained later.

When the deviation estimated by the prediction error analyzer 20 exceeds the predetermined first threshold and the fluctuation estimated by the fluctuation analyzer 30 exceeds the predetermined second threshold, the predictive sign detector 40 detects that the target has a predictive sign of a state transition.

The following is an embodiment of detecting a predictive sign of earthquake occurrence using the occurrence of an earthquake as the target state. In this case, the spatiotemporal data is the movement of the earth's surface over a wide area obtained from crustal movement observers using GNSS and seismometers in various regions.

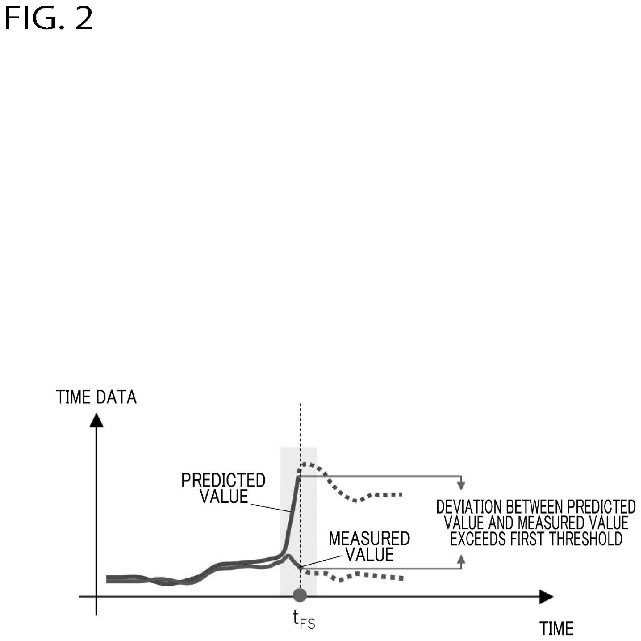

Figure 2 shows the time evolution of the predicted value and the measured value of spatiotemporal data. As shown in the figure, the magnitude of the deviation between the predicted value and the measured value increases rapidly from just before the time $t_{FS}$ and exceeds a predetermined first threshold at the time $t_{FS}$. This time, $t_{FS}$, is considered to be an important factor for predicting the occurrence of an earthquake.

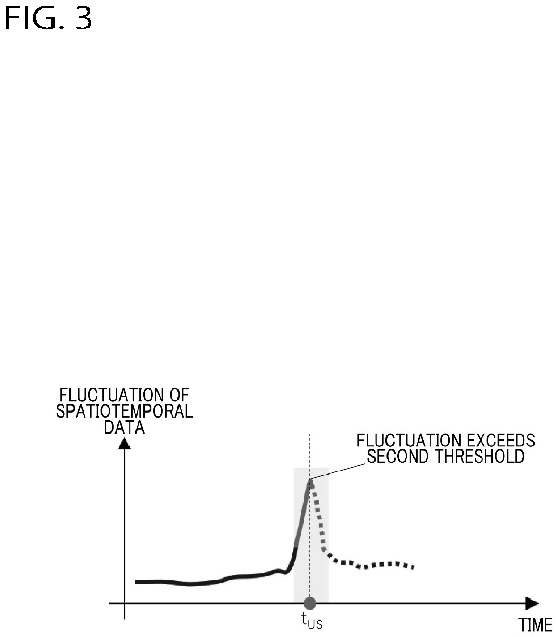

Figure 3 shows the time evolution of the fluctuation in the spatiotemporal data. As illustrated, the magnitude of the fluctuation in the spatiotemporal data increases rapidly just before the time $t_{US}$ and exceeds a predetermined second threshold at the time $t_{US}$. This time, $t_{US}$, is also considered to be an important factor for predicting the occurrence of an earthquake.

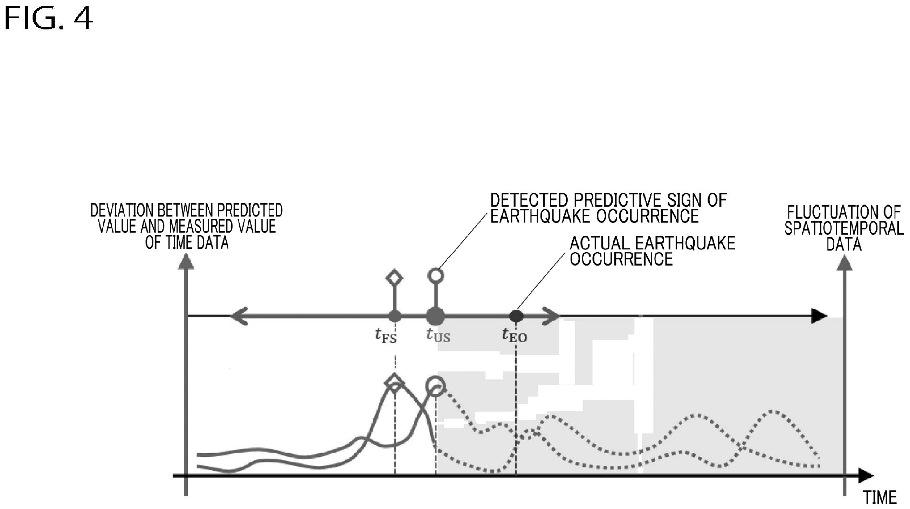

Figure 4 shows the timing of the detection of the predictive sign of the earthquake and the actual occurrence of the earthquake. As illustrated in the figure, in this example, the deviation between predicted value and the measured value first exceeds the first threshold at time $t_{FS}$, and then the fluctuation exceeds the second threshold at time $t_{US}$. The predictive sign detector 40 of the predictive sign detection device 1 detects the later time $t_{US}$ as the timing of the predictive sign of the earthquake occurrence, because both conditions, that the deviation exceeds the first threshold and that the fluctuation exceeds the second threshold, have been met. As illustrated in the figure, the earthquake actually occurs immediately after time $t_{US}$, indicating that the predictive sign of the earthquake occurrence was correctly detected. In this case, too, the predictive sign detector 40 detects the predictive sign of the target state transition because both of the two conditions, that the deviation exceeds the first threshold and that the fluctuation exceeds the second threshold, have been met.

In the above example, first the deviation between the predicted value and the measured value exceeded the first threshold at time $t_{FS}$, and then the fluctuation exceeded the second threshold at time $t_{US}$. However, depending on the type of target and the situation, the fluctuation may first exceed the second threshold at time $t_{FS}$ and then exceed the first threshold at time $t_{FS}$.

Thus, the technique of the present disclosure achieves the predictive sign detection of the state transition by integrating information obtained from the prediction error of spatiotemporal data and information obtained from fluctuation, and by detecting when both show a significant sign ,i.e., exceed a predetermined threshold value. Such a method of predictive sign detection is extremely novel and has not been disclosed or suggested in the conventional technology.

According to the embodiment, it is possible to accurately detect the timing of a predictive sign when the state of the target transitions.

In the following, unless otherwise noted, vectors and matrices are denoted by capital letters, e.g., vector V, matrix A, etc. Scalars are denoted by lower case letters, e.g., value x, etc.

The state of a k-dimensional dynamical system at a given time can be represented by k state variables. However, in actual experiments, it is not always possible to observe all k state variables, and more often than not, the number of dimensions of the original dynamical system is unknown. Therefore, we consider reconstructing attractors from time series data of the target variables.

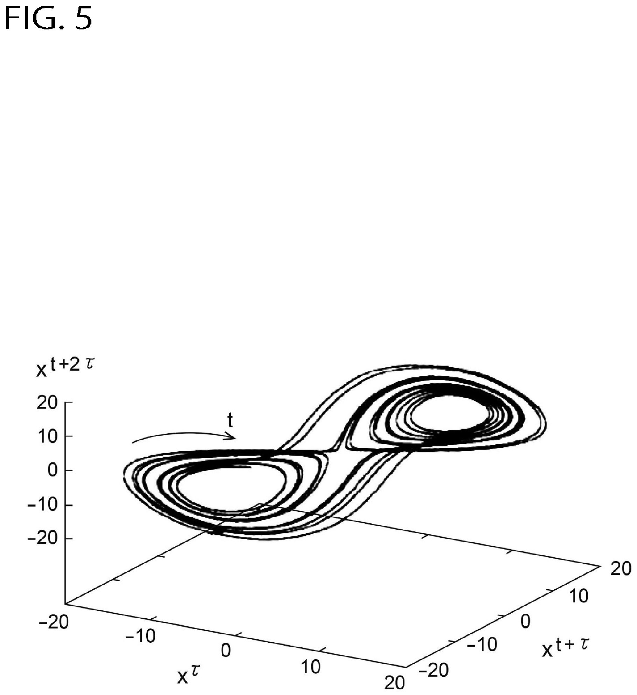

For this purpose, we use a transformation to the time-delayed coordinate system to extract n state variables from the measured time-series data $x^0$, $x^1$, ..., $x^{N-1}$ of the target variable at each appropriate delay time $\tau$, and we obtain an n-dimensional vector $V(t) = (x^t, x^{t+\tau}, ..., x^{t+(n-1)\tau})$ from them ($t=0, 1, ..., N-1-(n-1)\tau$). Here, for simplicity, we assume that $\tau = 1$ in the following. The time trajectory drawn by V(t) in n-dimensional space is the reconstructed attractor. Figure 5 shows the attractor reconstructed in this way.

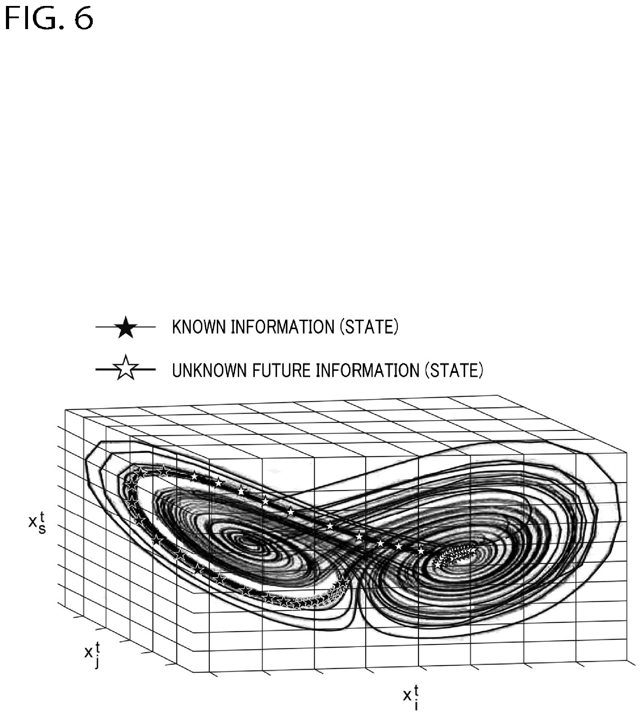

Alternatively, the attractor can be created by embedding non-time delays. That is, if the spatiotemporal data $X^t = x^t_1, x^t_2, ..., x^t_{\supset})$ ' of a multidimensional variable containing the time series of the target variable is obtained, By selecting some observation points, for example, $x^t_i, x^t_j, x^t_s$, the time trajectory drawn by the space vector $X^t$, i.e., $x^t_i, x^t_j, x^t_s$, of which components are the measured values of those observation points may be the reconstructed attractor, as illustrated in Figure 6. Here, D is the number of observation points and t = 1, 2, ..., m. The symbol ' denotes transposition of vectors (the same applies below.) According to embedding theory, if D > 2d > 0 and L > 2d > 0, these attractors can reconstruct dynamics that are topologically equivalent to the original system, i.e. Turkens' embedding theorem and its extension. See, for example, Non-Patent Literature 7 and 8. Here, d is the dimension of the original dynamical system or the box-count dimension of the attractors of the dynamical system.

Conversion from spatiotemporal data to temporal data of the target variable

The following relationship holds between the space vector $X^t$ and the time-delayed coordinate vector $Y^t$ of the target variable. The transformation expressed in this equation is sometimes referred to as "spatiotemporal information transformation."

[Equation 1]

$$\begin{cases} \Phi(X^t) = Y^t, \\ X^t = \Psi(Y^t), \end{cases} \quad (1)$$

Here, $\Phi$ is a differentiable nonlinear transformation from a D-dimensional vector space to an L-dimensional vector space. L is the dimension of the time-delay coordinate vector $Y^t$. $\Psi$ is a differentiable nonlinear transformation from L-dimensional vector space to D-dimensional vector space. $\Phi$ and $\Psi$ = I, i.e., the composite transformation of $\Phi$ and $\Psi$ is the identity transformation.

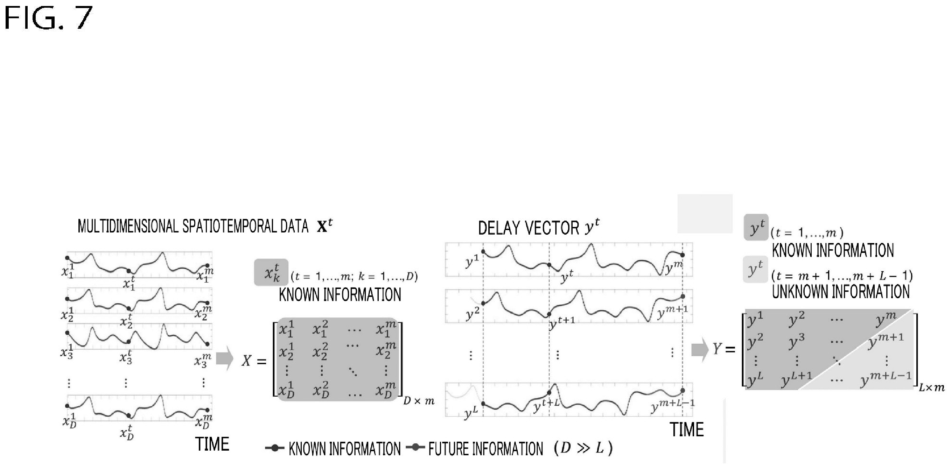

For a given number m, if m states $x^t$, t = 1, 2, ..., m, are measured, at t = m, the target variable y has L-1 unknown future values { $y^{m+1}$, $y^{m+2}$, ..., $y^{m+L-1}$} exist. Figure 7 shows schematically the vector of multidimensional spatiotemporal data and target variables.

It is difficult to find such nonlinear transformations $\Phi$ and $\Psi$ in practice. Therefore, for example, we linearize the nonlinear transformations $\Phi$ and $\Psi$ as follows, t = 1, 2, ..., m.

[Equation 2]

$$\begin{cases} AX^t = Y^t, \\ X^t = BY^t, \end{cases} \quad (2)$$

Here, AB = E. A is an L x D matrix, B is a D x L matrix and E is an L x L unit matrix. D and L, representing the number of rows and columns, are capitalized but scalar, as below. By solving equation (2) for A and B, the obtained coefficients can be used to obtain L-1 unknown future values { $y^{m+1}$ $y^{m+2}$, ..., $y^{m+L-1}$ }.

Autoreservoir

Alternatively, the accuracy, robustness and computational efficiency of the prediction can be greatly improved by using a neural network to construct a nonlinear transformation F as in equation (3), based on equation (2) of the spatiotemporal information transformation.

As an example, a multi-layer feed-forward neural network F can be applied to the neural network section. Here, the weights between neurons are given randomly in advance.

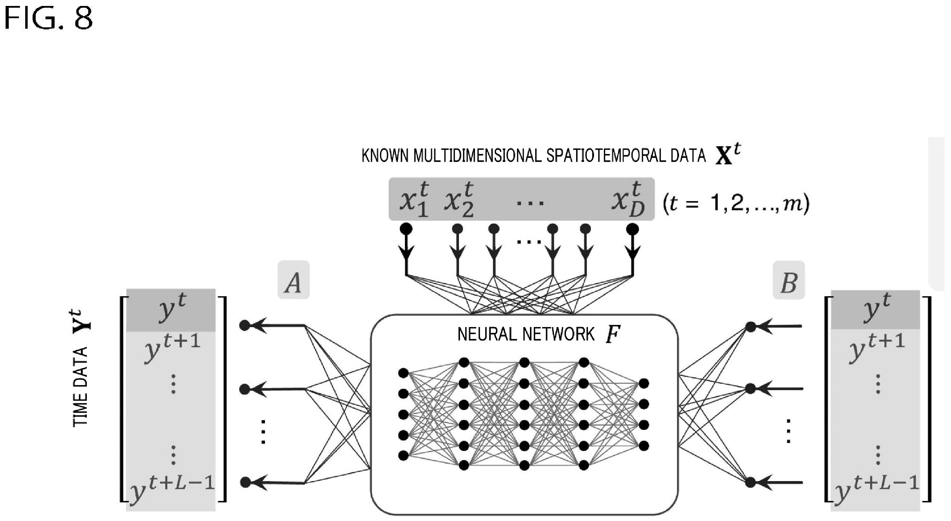

Figure 8 shows a schematic example of an autoreservoir neural network F. This neural network includes three intermediate layers and uses the hyperbolic tangent, tanh, as the activation function. However, it is not limited to this, and any suitable layer, activation function or network structure may be used.

Throughout the entire neural network F process, the original D variables $X^t = (x^t_1, x^t_2, ..., x^t_D)'$ are converted to D~ variables $F(X^t) = (F_1(X^t), F_2(X^t), ..., F_{D~}(X^-))'$. The input $X^t$ and the output $F(X^t)$ time evolves. In other words, this method makes the dynamics of the measured high-dimensional data $X^t$ a reservoir. This is in contrast to conventional reservoir computing, where the reservoir is the dynamics of the built-in randomly coupled recurrent neural network. In other words, the autoreservoir neural network can be represented by the following autoreservoir neural network-based spatiotemporal transformation equations, t = 1, 2, ..., m.

[Equation 3]

$$\begin{cases} AF(X^t) = Y^t, \\ F(X^t) = BY^t, \end{cases} \quad (3)$$

Here, A is the L x D~ matrix, B is the D~ x L matrix, AB = I, I is the L x L unit matrix. F is a nonlinear transformation, which is constructed by a neural network. D and D~ may be different. A and B are two weight matrices determined based on measured data.

Given X and F, the future values of the target variables $\{y^{m+1}, y^{m+2}, ..., y^{m+L-1}\}$, along with the unknown weight matrices A and B, can be obtained by solving the autoreservoir neural network-based spatiotemporal transformation equation (3). In this way, multi-step future prediction is possible. Here, D > L is required generally.

Application to the embodiment

The spatiotemporal data of multidimensional variables indicating the state of the target, specifically, the n-dimensional spatiotemporal data measured at time t, acquired by the acquisition unit 10 of the predictive sign detection device 1 in this embodiment is denoted as vector $Z^t = (z^t_1, z^t_2, ..., z^t_n)'$. The matrix $(Z^1, Z^2, ..., Z^m)$ is constructed from the multidimensional data vectors measured at m times.

[Equation 4]

$$(Z^1, Z^2, \cdots, Z^m) = \begin{pmatrix} Z^1_1 & Z^2_1 & \cdots & Z^m_1 \\ Z^1_2 & Z^2_2 & \cdots & Z^m_2 \\ \vdots & \vdots & \ddots & \vdots \\ Z^1_n & Z^2_n & \cdots & Z^m_n \end{pmatrix} \quad (4)$$

First, based on the above embedding theory, the scalar variable v is transformed from the non-time-delayed coordinate space to the time-delayed coordinate space by an L-step delay-time embedding transformation. This yields a matrix $(V^1, V^2, ..., V^m)$ consisting of a time sequence vector $V^t = (v^t, v^{t+1}, ..., v^{t+L-1})'$ for m times, as follows.

[Equation 5]

$$(V^1, V^2, \cdots, V^m) = \begin{pmatrix} V^1 & V^2 & \cdots & V^m \\ V^1 & V^3 & \cdots & V^{m+1} \\ \vdots & \vdots & \ddots & \vdots \\ V^L & V^{L+1} & \cdots & V^{m+L-1} \end{pmatrix} \quad (5)$$

Here, the parameter L (>1) is the embedding dimension.

The following relationship holds between the spatial data vector $Z^t$ and the time sequence vector $V^t$, space-time transformation.

[Equation 6]

$$\begin{cases} \Phi(Z^t) = V^t, \\ Z^t = \Psi(V^t), \end{cases} \quad (6)$$

Here, $\Phi$ is a differentiable nonlinear transformation from n-dimensional vector space to L-dimensional vector space. $\Psi$ is a

differentiable nonlinear transformation from L-dimensional vector space to n-dimensional vector space. $\Phi$ and $\Psi$ = I, i.e., the composite transformation of $\Phi$ and $\Psi$ is the identity transformation. As mentioned above, if d is the dimension of the original dynamical system or the box-count dimension of the attractor, n > 2d > 0 and L > 2d > 0, then $V^t$ can be used to reconstruct dynamics that are topologically equivalent to the original system.

From the first equation in equation (6), it can be seen that $Z^t$ can be mapped to $V^t$. That is, the spatial information $(z^t_1, z^t_2, ..., z^t_n)'$ at one time t can be mapped to the temporal information $(v^t, v^{t+1}, ..., v^{t+L-1})'$ of one variable at multiple times $\{t, t+1, ..., t+L-1\}$. Thus, the dynamics of an n-dimensional system can be represented by data in one variable.

In general, the states and phenomena that this disclosure deals with are nonlinear and their temporal changes are non-stationary. In this case, it is considered that time series data in the immediate short term contain more reliable information about the dynamics in the near future than time series data in the distant past. Therefore, the use of high-dimensional short-term data, e.g., data within a sliding time window, based on the spatiotemporal transformation equation (6) is expected to reduce problems caused by nonlinearities and non-stationarity. However, it is difficult to estimate nonlinear transformations $\Phi$ and $\Psi$ using only short-term time series data.

By constructing the nonlinear transformation F with a neural network, the equation (6) of the spatiotemporal transformation can be approximated to a simpler form (7).

[Equation 7]

$$\begin{cases} PF(Z^t) = V^t, \\ P(Z^t) = QV^t, \end{cases} \quad (7)$$

Here, P is an L x n~ matrix, Q is an n~ x L matrix, and PQ = E. E is an L x L unit matrix. F = $(F_1, F_2, ..., F_{n\sim})$ is a nonlinear transformation, each $F_k$ is a transformation from n-dimensional vector space to real number space.

The autoreservoir F is actually a multilayer neural network built from connected nodes, in which the weights are given randomly and are fixed in advance, rather than being learned, in the computation. Thus, in contrast to equation (6), equation (7) can be solved efficiently.

As explained above, the transformation from spatiotemporal data to time data of the target variable may be a transformation by embedding in non-time-delayed coordinate space or in time-delayed coordinate space.

The transformation from spatiotemporal data to time data of the target variable may be computed using machine learning. In particular, machine learning may be machine learning using the autoreservoir neural network described above.

The fluctuation analyzer 30 may calculate the fluctuation of the state of the target by converting to time data of virtual variables based on spatiotemporal data. In particular, the virtual variables may be data representing the movement of the target state on the central manifold.

Dynamical Network Markers

When a system transitions from one stable state to another stable by causing a bifurcation, there is a particular group of variables that satisfies the following three properties immediately before the transition.

1. the standard deviation of the variables in the group rises sharply as the transition approaches.
2. when approaching a transition, the absolute value of the correlation coefficient between variables in the group rises sharply.
3. as the transition is approached, the absolute value of the correlation between variables within the group and variables outside the group rapidly decreases.

At this point, this variable is called a dynamic network marker (DNM). By paying particular attention to this group in the system, we can efficiently perform predictive sign detection. As an indicator of this, we define the dynamic network marker score I as follows.

[Equation 8]

$$I = \frac{SD_d \cdot PCC_d}{PCC_O} \quad (8)$$

Here, $SD_d$ is the average of the standard deviations of all variables included in the dynamic network marker, $PCC_d$ is the average of the absolute values of the correlation coefficients of all pairs between variables included in the dynamic network marker, $PCC_O$ is the average of the absolute values of the correlation coefficients of all pairs between variables included and not included in the dynamic network marker. $PCC_O$ may be deleted and replaced by the value 1.

From the above three properties, it is expected that the numerator of the score I increases, and the denominator decreases as the transition is approaching, and that the overall score I value increases rapidly just before the transition. Therefore, by observing this, it is thought that the predictive sign can be detected.

In this theory, the equation describing the system is linearly approximated near the equilibrium point and the behavior of the dominant eigenvector direction of its Jacobian matrix is the focus of attention. According to this, for each variable $x_i$ of the system, if the value of the corresponding component of the dominant eigenvector of the Jacobian matrix immediately before the transition is not zero, it is included in the dynamic network marker, and if it is zero, it is not included in the dynamic network marker, and thus three properties of dynamic network markers are derived. For more details on dynamic network markers, see, for example, Non-Patent Literatures 9 and 10.

Applying the above dynamic network markers, in one embodiment, the fluctuation analyzer 30 may estimate the fluctuation of spatiotemporal data using the dynamic network markers. This allows for efficient and accurate estimation of spatiotemporal data fluctuation.

In one embodiment, the fluctuation analyzer 30 may start calculating a fluctuation when the deviation estimated by the prediction error analyzer 20 exceeds a predetermined first threshold value. This embodiment is particularly effective for objects where the deviation between the predicted value and the measured value of the time data in the spatiotemporal data occurs before the fluctuation.

In one embodiment, the transition of the state of the target may be the occurrence of an earthquake.

In one embodiment, the prediction sign of when the state of the subject will transition may be the occurrence of a pre-disease state, which is a state of transition from a normal state to a disease state.

In one embodiment, the transformation from non-time-delayed coordinate space to time-delayed coordinate space may be expressed as follows.

$$\Phi(Z^t) = V^t$$

$$Z^t = \Psi(V^t)$$

$$\Phi \Psi = I$$

Here, Z is a vector at time t in non-time-delayed coordinate space, V is a vector at time t in time-delayed coordinate space, $\Phi$ and $\Psi$ are nonlinear transformations and I is an identity transformation.

In one embodiment, the transformation from non-time-delayed coordinate space to time-delayed coordinate space may be expressed as follows.

$$PF(Z^t) = V^t$$

$$F(Z^t) = QV^t$$

$$PQ = E$$

Here, F is a nonlinear transformation, P and Q are weight matrices and E is a unit matrix.

The second embodiment

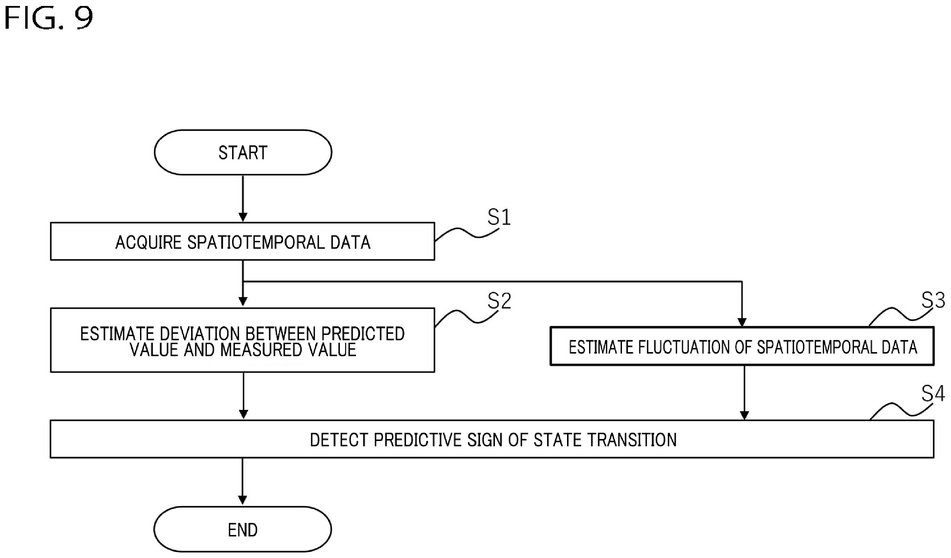

Figure 9 shows a flowchart showing the processing steps of a predictive sign detection method according to the second embodiment. This predictive sign detection method detects a predictive sign when the state of the target transitions. This predictive sign detection method includes an acquisition step S1 to acquire spatiotemporal data of multidimensional variables indicating the state of the target, a prediction error analysis step S2 to estimate the deviation between the predicted value and the measured value of the time data, a fluctuation analysis step S3 to estimate the fluctuation of the spatiotemporal data and a predictive sign detection step S4.

The prediction error analysis step S2 calculates predicted value by converting the spatiotemporal data of multidimensional variables into temporal data of the target variable, which is the variable to be predicted by the predictive sign detection method, and estimates the deviation between the predicted value and the measured value.

Fluctuation analysis step S3 calculates the fluctuation of the target state by fluctuation analysis using spatiotemporal data.

The predictive sign detection step S4 detects that there is a predictive sign of a state transition in the target when the deviation estimated in the prediction error analysis step S2 exceeds a predetermined first threshold and when the fluctuation estimated in the fluctuation analysis step S3 exceeds a predetermined second threshold.

According to this embodiment, it is possible to accurately detect the timing of the predictive sign when the state of the target is transitioning by following the procedure.

The third embodiment

The third embodiment is a program. This program causes a computer to execute the method including an acquisition step S1 to acquire spatiotemporal data of multidimensional variables indicating the state of the target, a prediction error analysis step S2 to estimate the deviation between the predicted value and the measured value of the time data, a fluctuation analysis step S3 to estimate the fluctuation of the spatiotemporal data and a predictive sign detection step S4.

The prediction error analysis step S2 calculates predicted value by converting the spatiotemporal data of multidimensional variables into temporal data of the target variable, which is the variable to be predicted by the predictive sign detection method, and estimates the deviation between the predicted value and the measured value.

Fluctuation analysis step S3 calculates the fluctuation of the target state by fluctuation analysis using spatiotemporal data.

The predictive sign detection step S4 detects that there is a predictive sign of a state transition in the target when the deviation estimated in the prediction error analysis step S2 exceeds a predetermined first threshold and when the fluctuation estimated in the fluctuation analysis step S3 exceeds a predetermined second threshold.

According to this embodiment, it is possible to implement as software a program that accurately detects the timing of the predictive sign when the state of the target is transitioning.

The fourth embodiment

The fourth embodiment is a recording medium. This recording medium records the program that causes a computer to execute the method including an acquisition step S1 to acquire spatiotemporal data of multidimensional variables indicating the state of the target, a prediction error analysis step S2 to estimate the deviation between the predicted value and the measured value of the time data, a fluctuation analysis step S3 to estimate the fluctuation of the spatiotemporal data and a predictive sign detection step S4.

The prediction error analysis step S2 calculates predicted value by converting the spatiotemporal data of multidimensional variables into temporal data of the target variable, which is the variable to be predicted by the predictive sign detection method, and estimates the deviation between the predicted value and the measured value.

Fluctuation analysis step S3 calculates the fluctuation of the target state by fluctuation analysis using spatiotemporal data.

The predictive sign detection step S4 detects that there is a predictive sign of a state transition in the target when the deviation estimated in the prediction error analysis step S2 exceeds a predetermined first threshold and when the fluctuation estimated in the fluctuation analysis step S3 exceeds a predetermined second threshold.

According to this embodiment, it is possible to record the program that accurately detects the timing of the predictive sign when the state of the target is transitioning.

Verification

To verify the effectiveness of the technology of the present disclosure, we applied spatiotemporal information transformation to multidimensional spatiotemporal data on wide-area movements of the earth's surface obtained from crustal movement instruments in various regions using GNSS and seismometers in various regions to detect a predictive sign of earthquake occurrence. As spatiotemporal data, we used data analyzed from earthquakes that occurred in five high-risk seismic zones/regions, i.e., Ibaraki Prefecture in Japan, west-central Hokkaido (around Iburi), Miyagi Prefecture, Sichuan Province in China and south-central Alaska in the United States.

The results are shown in Table 1.

Table 1

[Table 1]

| Region \ Indices | Monitoring years | Number of sliding windows* | Earthquake magnitude ($M$) | Number of earthquakes | TPR | FPR | Specificity | Accuracy | Average days ahead |
|---|---|---|---|---|---|---|---|---|---|
| All regions | | 10962 | $3.0 \leq M < 5.0$ | 1380 | 0.53 | 0.0100 | 0.9900 | 0.9321 | 6.08 |
| | | | $M \geq 5.0$ | 417 | 0.83 | 0.0098 | 0.9902 | 0.9841 | 6.23 |
| Ibaraki | 7 | 2557 | $3.0 \leq M < 5.0$ | 387 | 0.64 | 0.0065 | 0.9935 | 0.9402 | 6.13 |
| | | | $M \geq 5.0$ | 133 | 0.82 | 0.0107 | 0.9893 | 0.9804 | 6.23 |
| West-central Hokkaido | 6 | 2192 | $3.0 \leq M < 5.0$ | 100 | 0.45 | 0.0201 | 0.9799 | 0.9557 | 6.16 |
| | | | $M \geq 5.0$ | 52 | 0.81 | 0.0126 | 0.9874 | 0.9831 | 6.33 |
| Miyagi | 4 | 1465 | $3.0 \leq M < 5.0$ | 200 | 0.60 | 0.0126 | 0.9874 | 0.9338 | 5.02 |
| | | | $M \geq 5.0$ | 157 | 0.85 | 0.0084 | 0.9916 | 0.9761 | 6.02 |
| Sichuan | 5 | 1826 | $3.0 \leq M < 5.0$ | 322 | 0.34 | 0.0033 | 0.9967 | 0.8817 | 6.56 |
| | | | $M \geq 5.0$ | 14 | 0.93 | 0.0039 | 0.9961 | 0.9956 | 7.31 |
| South-central Alaska | 8 | 2922 | $3.0 \leq M < 5.0$ | 371 | 0.56 | 0.0074 | 0.9926 | 0.9381 | 6.35 |
| | | | $M \geq 5.0$ | 61 | 0.80 | 0.0112 | 0.9888 | 0.9849 | 6.44 |

\* The setting of sliding-window scheme of the observed time series was presented in Supplementary Note S2

As can be seen in Table 1, the technology of the present disclosure detected predictive signs of future earthquakes with an average accuracy of 93.21% and a specificity of 99.00% for 1380 earthquakes of magnitudes 3 to 5. For 417 strong earthquakes exceeding magnitude 5, the technique detected predictive signs of future earthquakes with an average accuracy of 98.41% and a specificity of 99.02%. Furthermore, for strong earthquakes exceeding magnitude 5, early warning signals could be identified an average of 6.23 days before the earthquake when based on daily GNSS data, and an average of 5.75 hours before the earthquake when based on 5-minute seismic waveform records.

As shown in Table 1, this verification showed that both TPR (True Positive Rate) and FPR (False Positive Rate) were able to stably detect predictive signs of earthquake occurrence, especially for strong earthquakes exceeding magnitude 5.

Table 2 compares existing methods, M1 to M10, and the technology of the present disclosure for detecting predictive signs of earthquakes occurring in the same areas as in Table 1.

Table 2

[Table 2]

| Methods / Region | Metrics | RSIT | M1 | M2 | M3 | M4 | M5 | M6 | M7 | M8 | M9 | M10 |
|---|---|---|---|---|---|---|---|---|---|---|---|---|
| Ibaraki | TPR | 0.82 | 0.49 | 0.62 | 0.16 | 0.58 | 0.74 | 0.45 | 0.55 | 0.69 | 0.27 | 0.68 |
| | Aggregative score | 0.87 | 0.62 | 0.69 | 0.49 | 0.65 | 0.71 | 0.62 | 0.61 | 0.72 | 0.58 | 0.70 |
| West-central Hokkaido | TPR | 0.81 | 0.56 | 0.63 | 0.40 | 0.44 | 0.54 | 0.39 | 0.67 | 0.68 | 0.33 | 0.58 |
| | Aggregative score | 0.80 | 0.61 | 0.65 | 0.53 | 0.56 | 0.60 | 0.51 | 0.62 | 0.68 | 0.58 | 0.63 |
| Miyagi | TPR | 0.85 | 0.72 | 0.62 | 0.55 | 0.48 | 0.66 | 0.68 | 0.74 | 0.64 | 0.64 | 0.63 |
| | Aggregative score | 0.92 | 0.81 | 0.77 | 0.65 | 0.65 | 0.78 | 0.78 | 0.83 | 0.77 | 0.73 | 0.75 |
| Sichuan | TPR | 0.93 | 0.21 | 0.29 | 0.36 | 0.50 | 0.36 | 0.50 | 0.57 | 0.64 | 0.21 | 0.57 |
| | Aggregative score | 0.86 | 0.43 | 0.49 | 0.45 | 0.52 | 0.50 | 0.56 | 0.55 | 0.59 | 0.43 | 0.55 |
| South-central Alaska | TPR | 0.80 | 0.54 | 0.59 | 0.49 | 0.31 | 0.51 | 0.62 | 0.59 | 0.61 | 0.43 | 0.59 |
| | Aggregative score | 0.80 | 0.58 | 0.63 | 0.52 | 0.46 | 0.59 | 0.63 | 0.61 | 0.65 | 0.63 | 0.59 |

* Aggregative score is the average of TPR, precision and accuracy.

The existing methods listed in Table 2 are as follows.

Method 1 (M1): M1 is a real-time anomaly detection method based on a window-based prediction model.

Method 2 (M2): M2 is a supervised machine learning algorithm for GNSS positioning time series prediction.

Method 3 (M3): M3 is a method based on the randomness of outliers in GPS time series.

Method 4 (M4): M4 is GPS Interactive Time Series Analysis, GITSA, software program developed for visualization and analysis of GPS time series in geodesy and geodynamics research.

Method 5 (M5): M5 is an artificial neural network, ANN, for earthquake warning using real-time GNSS data.

Method 6 (M6): M6 is a deep learning algorithm called WANEH. This method detects anomalies in time series data by combining wavelets with neural networks and Hilbert transforms.

Method 7 (M7): M7 is a method based on martingale theory, which extracts anomalies as precursors to earthquakes from a series of GPS data.

Method 8 (M8): M8 is a graphical method for detecting outliers in time series data of routine measurements.

Method 9 (M9): M9 is support vector regression, SVR. It is one of the supervised machine learning algorithms.

Method 10 (M10): M10 is delayed long short-term memory, dLSTM. This is one of the methods for anomaly detection in time series data.

As can be seen from Table 2, the technique of the present disclosure can detect predictive sign of earthquake occurrence with higher accuracy than existing earthquake prediction methods.

Integration of disease prevention and pre-disease medical science

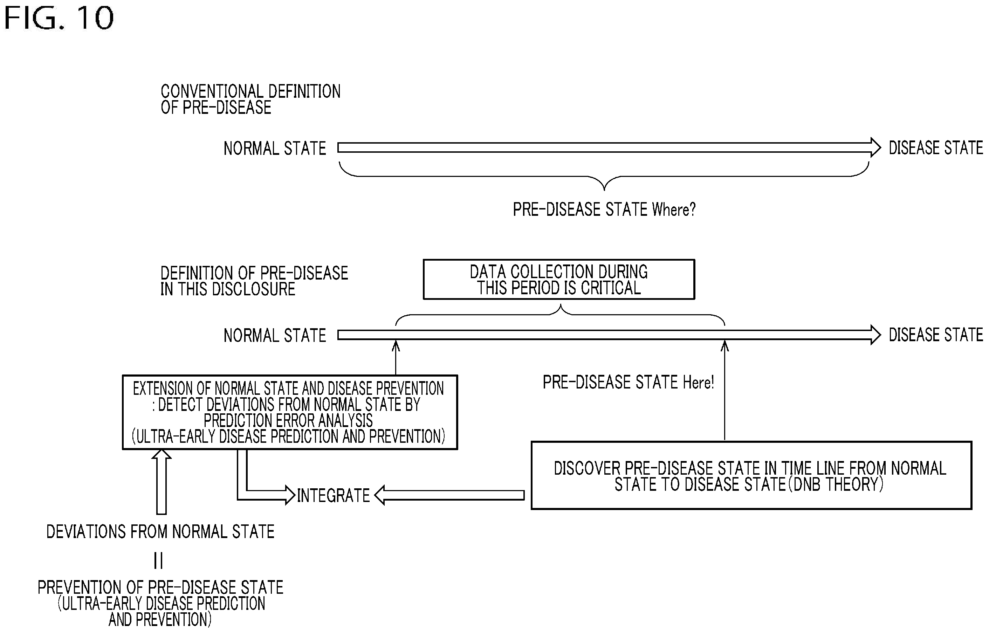

One of the remarkable effects expected to be realized by this technology is the integration of disease prevention and pre-disease medical science. Figure 10 is a schematic diagram explaining the definition of pre-disease state. The upper row shows the conventional definition of pre-disease state, and the lower row shows the definition of pre-disease state in the present disclosure. Conventionally, the pre-disease state was considered to lie between the normal state and the disease state, however it was not always clear when exactly the pre-disease state was defined. In contrast, in the present disclosure, by collecting data for the period of time leading up to the pre-disease state, it is possible to accurately capture a sign of transition from the normal state to the pre-disease state. This makes it possible to integrate very early disease prediction and prevention, i.e., healthy life extension and disease prevention, where deviations from the normal state can be detected by prediction error analysis, and DNB theory, i.e., detecting the pre-disease state in the time span from the normal state to the disease state. See, for example, Non-Patent literature 10.

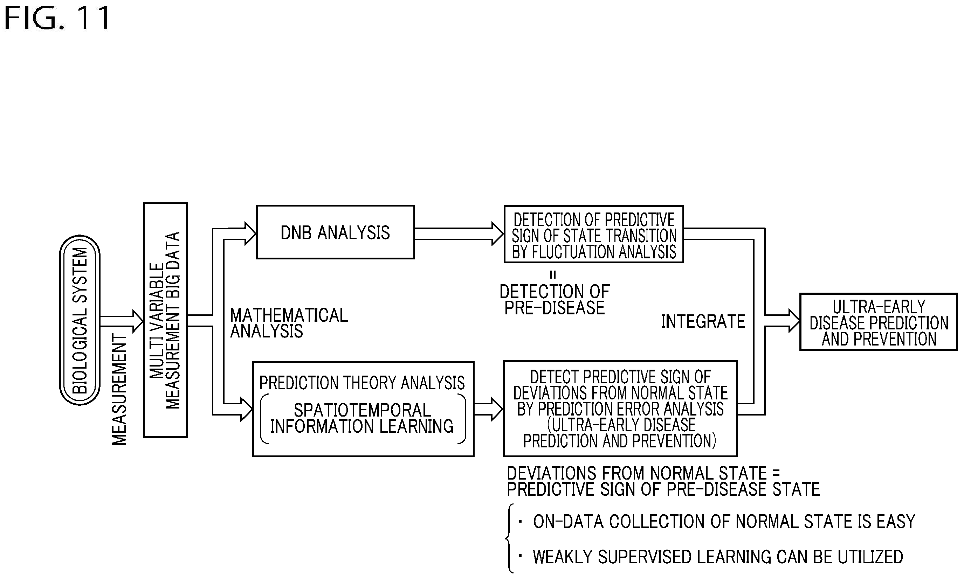

Figure 11 shows a schematic diagram of the integration of DNB theory analysis and prediction theory analysis. Both DNB theory analysis and prediction theory analysis collect multivariate measurement big data by measuring biological systems. However, DNB theory analysis uses fluctuation analysis to detect predictive a sign of state transition. This enables detection of pre-disease state. On the other hand, prediction theory analysis, i.e., spatial-temporal information transformation learning, detects a sign of deviation from the normal state through prediction error analysis. This enables early disease prediction and prevention. The technology of the present disclosure opens the way to very early disease prediction and prevention, which was not possible before, by integrating DNB theory analysis and prediction theory analysis.

The above description is based on examples of the invention. It is understood by those skilled in the art that these examples are illustrative, that various variations are possible in the combination of each component and each processing process, and that such variations are also within the scope of the invention.

In understanding the technical concept abstracted from the embodiments and variations, the technical concept should not be interpreted as limited to the contents of the embodiments and variations. The aforementioned embodiments and variations are merely concrete examples, and many design changes, such as changes, additions, and deletions of components, are possible. In the embodiments, the contents where such design changes are possible are emphasized with the notation "embodiments". However, design changes are also permitted for contents without such notation.

INDUSTRIAL APPLICABILITY

The technology of the present disclosure can be used for forecasting in a wide range of natural and social fields, such as earthquake prediction, early detection and early treatment of diseases, development of new drugs and medical devices, prediction of traffic congestion, prediction of sudden climate changes, prediction of stock price fluctuations, and prediction of political changes.

REFERENCE SIGNS LIST

1 Predictive sign detection device,
10 Acquisition unit,
20 Prediction error analyzer,
30 Fluctuation analyzer,
40 Predictive sign detector,
S1 Acquisition step,
S2 Prediction error analysis step,
S3 Fluctuation analysis step,
S4 Predictive sign detection step.

**Claims**

1. A predictive sign detection device for detecting a predictive sign of a transition in the state of a target, comprising:

    an acquisition unit that acquires spatiotemporal data of multidimensional variables indicating the state of the target;
    a prediction error analyzer that estimates the deviation between the predicted value and the measured value of the time data in the spatiotemporal data;
    a fluctuation analyzer that estimates a fluctuation in spatiotemporal data; and
    a predictive sign detector,
    wherein the prediction error analyzer calculates the predicted value by converting the spatiotemporal data of multidimensional variables into the time data of the target variable, which is the variable to be predicted by the predictive sign detection device, and estimates the deviation between the predicted value and the measured value,
    wherein the fluctuation analyzer calculates the fluctuation of the target state by fluctuation analysis using the spatiotemporal data and
    wherein the predictive sign detector detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

2. The predictive sign detection device according to claim 1,
    wherein the transformation from spatiotemporal data to time data of the target variable is a transformation by embedding in non-time-delayed coordinate space or in time-delayed coordinate space.

3. The predictive sign detection device according to claim 2,

wherein the transformation from spatiotemporal data to time data of the target variable is computed using machine learning.

4.  The predictive sign detection device according to claim 1,
    wherein the fluctuation analyzer calculates the fluctuation of the state of the target by converting to time data of virtual variables based on spatiotemporal data.

5.  The predictive sign detection device according to claim 4,
    wherein the virtual variables are data representing the movement of the target state on the central manifold.

6.  The predictive sign detection device according to claim 1,
    wherein the fluctuation analyzer estimates the fluctuation of spatiotemporal data using the dynamic network markers.

7.  The predictive sign detection device according to claim 6,
    wherein the fluctuation analyzer calculates fluctuation when the deviation estimated by the prediction error analyzer exceeds a predetermined first threshold value.

8.  The predictive sign detection device according to claim 1,
    wherein the transition of the state of the target is the occurrence of an earthquake.

9.  The predictive sign detection device according to claim 1,
    wherein the prediction sign of when the state of the subject will transition is the occurrence of a pre-disease state, which is a state of transition from a normal state to a disease state.

10. The predictive sign detection device according to claim 3,
    wherein the transformation from non-time-delayed coordinate space to time-delayed coordinate space is expressed as follows,

$$\Phi(Z^t) = V^t,$$

$$Z^t = \Psi(V^t),$$

$$\Phi\,\Psi = I,$$

wherein Z is a vector at time t in non-time-delayed coordinate space,
wherein V is a vector at time t in time-delayed coordinate space,
wherein $\Phi$ and $\Psi$ are nonlinear transformations and
wherein I is an identity transformation.

11. The predictive sign detection device according to claim 10,
    wherein the transformation from non-time-delayed coordinate space to time-delayed coordinate space is expressed as follows,

$$PF(Z^t) = V^t,$$

$$F(Z^t) = QV^t,$$

$$PQ = E,$$

wherein F is a nonlinear transformation,
wherein P and Q are weight matrices and
wherein E is a unit matrix.

12. A predictive sign detection method for detecting a predictive sign of a transition in the state of a target, comprising:

an acquisition step to acquire spatiotemporal data of multidimensional variables indicating the state of the target;
a prediction error analysis step to estimate the deviation between the predicted value and the measured value of the time data;
a fluctuation analysis step to estimate the fluctuation of the spatiotemporal data; and
a predictive sign detection step,
wherein the prediction error analysis step calculates the predicted value by converting the spatiotemporal data of multidimensional variables into the time data of the target variable, which is the variable to be predicted by the predictive sign detection device, and estimates the deviation between the predicted value and the measured value,
wherein the fluctuation analysis step calculates the fluctuation of the target state by fluctuation analysis using the spatiotemporal data and
wherein the predictive sign detection step detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

13. A program for causing a computer to execute a method for detecting a predictive sign of a transition in the state of a target, comprising:

an acquisition step to acquire spatiotemporal data of multidimensional variables indicating the state of the target;
a prediction error analysis step to estimate the deviation between the predicted value and the measured value of the time data;
a fluctuation analysis step to estimate the fluctuation of the spatiotemporal data; and
a predictive sign detection step,
wherein the prediction error analysis step calculates the predicted value by converting the spatiotemporal data of multidimensional variables into the time data of the target variable, which is the variable to be predicted by the predictive sign detection device, and estimates the deviation between the predicted value and the measured value,
wherein the fluctuation analysis step calculates the fluctuation of the target state by fluctuation analysis using the spatiotemporal data and
wherein the predictive sign detection step detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

14. A recording medium containing a program for causing a computer to execute a method for detecting a predictive sign of a transition in the state of a target, comprising:

an acquisition step to acquire spatiotemporal data of multidimensional variables indicating the state of the target;
a prediction error analysis step to estimate the deviation between the predicted value and the measured value of the time data;
a fluctuation analysis step to estimate the fluctuation of the spatiotemporal data; and
a predictive sign detection step,
wherein the prediction error analysis step calculates the predicted value by converting the spatiotemporal data of multidimensional variables into the time data of the target variable, which is the variable to be predicted by the predictive sign detection device, and estimates the deviation between the predicted value and the measured value,
wherein the fluctuation analysis step calculates the fluctuation of the target state by fluctuation analysis using the spatiotemporal data and
wherein the predictive sign detection step detects that there is a predictive sign of a transition in the state of the target when the deviation estimated by the prediction error analyzer exceeds a predetermined first threshold value and the fluctuation estimated by the fluctuation analyzer exceeds a predetermined second threshold value.

# FIG. 1

SPATIOTEMPORAL DATA

1

PREDICTIVE SIGN DETECTION DEVICE 10

ACQUISITION UNIT

SPATIOTEMPORAL DATA

SPATIOTEMPORAL DATA

20

PREDICTION ERROR ANALYZER

30

FLUCTUATION ANALYZER

DEVIATION BETWEEN PREDICTED VALUE AND MEASURED VALUE OF SPATIOTEMPORAL DATA

FLUCTUATION OF SPATIOTEMPORAL DATA

40

PREDICTIVE SIGN DETECTOR

PREDICTIVE SIGN

# FIG. 2

TIME DATA

PREDICTED
VALUE

MEASURED
VALUE

DEVIATION BETWEEN PREDICTED
VALUE AND MEASURED VALUE
EXCEEDS FIRST THRESHOLD

$t_{FS}$

TIME

# FIG. 3

FLUCTUATION OF SPATIOTEMPORAL DATA

FLUCTUATION EXCEEDS SECOND THRESHOLD

$t_{US}$

TIME

EP 4 752 758 A1

# FIG. 4

DEVIATION BETWEEN PREDICTED VALUE AND MEASURED VALUE OF TIME DATA

DETECTED PREDICTIVE SIGN OF EARTHQUAKE OCCURRENCE

ACTUAL EARTHQUAKE OCCURRENCE

FLUCTUATION OF SPATIOTEMPORAL DATA

$t_{FS}$   $t_{US}$   $t_{EO}$

TIME

# FIG. 5

FIG. 6

KNOWN INFORMATION (STATE)

UNKNOWN FUTURE INFORMATION (STATE)

$x_s^t$

$x_j^t$

$x_i^t$

# FIG. 7

FIG. 8

KNOWN MULTIDIMENSIONAL SPATIOTEMPORAL DATA $\mathbf{X}^t$

$x_1^t \quad x_2^t \quad \cdots \quad x_D^t \quad (t = 1, 2, \ldots, m)$

$A$

$B$

NEURAL NETWORK $F$

TIME DATA $\mathbf{Y}^t$

$\begin{bmatrix} y^t \\ y^{t+1} \\ \vdots \\ \vdots \\ y^{t+L-1} \end{bmatrix}$

$\begin{bmatrix} y^t \\ y^{t+1} \\ \vdots \\ \vdots \\ y^{t+L-1} \end{bmatrix}$

FIG. 9

```
                    ┌──────────────┐
                    │    START     │
                    └──────┬───────┘
                           │
                           ▼
        ┌─────────────────────────────────────┐  S1
        │    ACQUIRE SPATIOTEMPORAL DATA       │
        └──────┬──────────────────────────────┘
               │                         │
               ▼                         ▼
┌─────────────────────────────┐  S2   ┌──────────────────────────────────────┐  S3
│ ESTIMATE DEVIATION BETWEEN  │       │ ESTIMATE FLUCTUATION OF SPATIOTEMPORAL │
│  PREDICTED VALUE AND        │       │            DATA                        │
│  MEASURED VALUE             │       │                                        │
└──────────┬──────────────────┘       └──────────────────┬─────────────────────┘
           │                                              │
           ▼                                              ▼
┌────────────────────────────────────────────────────────────────────────────┐  S4
│              DETECT PREDICTIVE SIGN OF STATE TRANSITION                      │
└──────────┬───────────────────────────────────────────────────────────────────┘
           │
           ▼
    ┌──────────────┐
    │     END      │
    └──────────────┘
```

EP 4 752 758 A1

# FIG. 10

CONVENTIONAL DEFINITION
OF PRE-DISEASE

NORMAL STATE ⟶ DISEASE STATE

PRE-DISEASE STATE Where?

DEFINITION OF PRE-DISEASE
IN THIS DISCLOSURE

DATA COLLECTION DURING
THIS PERIOD IS CRITICAL

NORMAL STATE ⟶ DISEASE STATE

PRE-DISEASE STATE Here!

EXTENSION OF NORMAL STATE AND DISEASE PREVENTION
: DETECT DEVIATIONS FROM NORMAL STATE BY
PREDICTION ERROR ANALYSIS
(ULTRA-EARLY DISEASE PREDICTION AND PREVENTION)

DISCOVER PRE-DISEASE STATE IN TIME LINE FROM NORMAL
STATE TO DISEASE STATE (DNB THEORY)

INTEGRATE

DEVIATIONS FROM NORMAL STATE
||
PREVENTION OF PRE-DISEASE STATE
(ULTRA-EARLY DISEASE PREDICTION
AND PREVENTION)

EP 4 752 758 A1

FIG. 11

FIG. 11

BIOLOGICAL SYSTEM

MEASUREMENT

MULTI VARIABLE MEASUREMENT BIG DATA

DNB ANALYSIS

MATHEMATICAL ANALYSIS

PREDICTION THEORY ANALYSIS

SPATIOTEMPORAL INFORMATION LEARNING

DETECTION OF PREDICTIVE SIGN OF STATE TRANSITION BY FLUCTUATION ANALYSIS

=

DETECTION OF PRE-DISEASE

DETECT PREDICTIVE SIGN OF DEVIATIONS FROM NORMAL STATE BY PREDICTION ERROR ANALYSIS (ULTRA-EARLY DISEASE PREDICTION AND PREVENTION)

DEVIATIONS FROM NORMAL STATE = PREDICTIVE SIGN OF PRE-DISEASE STATE

· ON-DATA COLLECTION OF NORMAL STATE IS EASY

· WEAKLY SUPERVISED LEARNING CAN BE UTILIZED

INTEGRATE

ULTRA-EARLY DISEASE PREDICTION AND PREVENTION

EP 4 752 758 A1

## INTERNATIONAL SEARCH REPORT

| | |
|---|---|
| International application No. | |
| | **PCT/JP2024/018720** |

| | |
|---|---|
| **A.** | **CLASSIFICATION OF SUBJECT MATTER** |

*G06F 17/15*(2006.01)i; *G06Q 10/04*(2023.01)i; *G16B 40/00*(2019.01)i
FI:   G06F17/15; G06Q10/04; G16B40/00

According to International Patent Classification (IPC) or to both national classification and IPC

| | |
|---|---|
| **B.** | **FIELDS SEARCHED** |

Minimum documentation searched (classification system followed by classification symbols)

G06F17/15; G06Q10/04; G16B40/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| A | JP 2020-020707 A (EARTHQUAKE PREDICTION RES CENTER) 06 February 2020 (2020-02-06)<br>    paragraphs [0008], [0015] | 1-14 |
| A | 村田健哉、春木孝之、吉田泰彦、奥 牧人、小泉桂一、門脇 真. デン ゲ熱ウ イ ル ス感染経過データに対する動的ネットワークバイオマーカー解析の適用. 情報処理学会研究報告. 05 March 2020, vol. 2000-BIO-61, no. 2, pp. 1-6, ISSN 2188-8590, (MURATA, Kenya. HARUKI, Takayuki. YOSHIDA, Yasuhiko. OKU, Makito. KOIZUMI, Keiichi. KADOWAKI, Makoto. Application of dynamical network biomarker analysis to dengue fever virus infection data. IPSJ SIG Technical Report.)<br>    abstract | 1-14 |
| A | JP 2020-034340 A (JAPAN EARTHQUAKE SCIENCE EXPLOR AGENCY) 05 March 2020 (2020-03-05)<br>    paragraphs [0039]-[0045], fig. 13 | 1-14 |

☐ Further documents are listed in the continuation of Box C.       ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **10 July 2024** | **23 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/018720**

| Patent document cited in search report | | | Publication date (day/month/year) | Patent family member(s) | Publication date (day/month/year) |
|---|---|---|---|---|---|
| JP | 2020-020707 | A | 06 February 2020 | (Family: none) | |
| JP | 2020-034340 | A | 05 March 2020 | JP 6438169 B1 paragraphs [0039]-[0045], fig. 13 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014064515 A
- JP 2014083194 A
- WO 2018207925 A

**Non-patent literature cited in the description**

- *Nature*, 2005, vol. 434, 777-782
- *Nature Medicine*, 2003, vol. 9, 241-242
- **CORESH, J.** Transition models for change-point estimation in logistic regression. *Statistics in Medicine*, 2003, vol. 22 (7), 1141-1162
- **PAEK, S.H.** ; **CHUNG, H.-T** ; **JEONG, S.S.** ; **PARK, C.-K** ; **KIM, C.-Y** ; **KIM, J.E.** ; **KIM D.G.** ; **JUNG, H.-W.** Hearing preservation after gamma knife stereotactic radiosurgery of vestibular schwannoma. *Cancer*, 2005, vol. 104, 580-590
- **LIU, J.K.** ; **ROVIT, R.L** ; **COULDWELL, W.T.** *Pituitary Apoplex*
- **TANAKA, G.** ; **TSUMOTO, K.** ; **TSUJI, S** ; **AIHARA, K.** Bifurcation analysis on a hybrid systems model of intermittent hormonal therapy for prostate cancer. *Physica Dermatol. Physica D: Nonlinear Phenomena*, 2008, vol. 237, 2616-2627
- **TAKENS, F.** Dynamical Systems and Turbulence.. *Lecture Notes in Mathematics*, 1981, vol. 898, 366-381
- **SAUER, T.** ; **YORKE, J.A** ; **CASDAGLI, M.** Embedology. *J. Stat. Phys.*, 1991, vol. 65, 579-615
- **SAUER, T.** ; **YORKE, J.A.** ; **CASDAGLI, M**. Embedology. *J. Stat. Phys.*, 1991, vol. 65, 579-615
- **CHEN, L.** ; **LIU, R.** ; **LIU, Z.-P., LI, M.** ; **AIHARA K.** Detecting early-warning signals for sudden deterioration of complex diseases by dynamical network biomarkers. *Scientific Reports*, 2012, vol. 2